# EUROPEAN PATENT SPECIFICATION

(11) **EP 0 981 629 B1**
(45) Date of publication and mention of the grant of the patent: **13.08.2008**
(21) Application number: 98918814.9
(22) Date of filing: 28.04.1998
(51) Int. Cl.: C12N 15/57, C12N 9/64, C12N 5/10, G01N 33/573, C07K 16/40, C12N 5/24, C12Q 1/68, C12Q 1/37

(54) **FORMS OF PROSTATE SPECIFIC ANTIGEN AND METHODS FOR THEIR DETECTION**
PROSTAT-SPEZIFISCHES-ANTIGENFORMEN UND VERFAHREN ZU IHRER BESTIMMUNG
FORMES D'ANTIGENE A SPECIFICITE PROSTATIQUE (ASP) ET LEURS PROCEDES DE DETECTION

(30) Priority: 30.04.1997 US 846408
(43) Date of publication of application: 01.03.2000
(73) Proprietor: HYBRITECH INCORPORATED, San Diego, CA 92196-9006 (US)
(72) Inventor: SAEDI, Mohammad, S., San Diego, CA 92126 (US); MIKOLAJCZYK, Stephen, D., San Diego, CA 92117 (US); KUMAR, Abhay, San Diego, CA 92129 (US); GRAUER, Lana, S., San Diego, CA 92130 (US); WOLFERT, Robert, L., San Diego, CA 92131 (US); RITTENHOUSE, Harry, G., Del Mar, CA 92014 (US)
(74) Representative: Thomson, Craig Richard
(86) International application number: PCT/US1998/008498
(87) International publication number: WO 1998/049323

(56) References cited:
- EP-A- 0 725 139
- EP-A- 0 843 006
- WO-A-94/10343
- WO-A-96/34964
- KUMAR A ET AL: "Expression of human glandular kallikrein, hK2, in mammalian cells" CANCER RESEARCH, vol. 56, 1 December 1996, pages 5397-5402, XP002074988 cited in the application
- KURKELA R ET AL: "EXPRESSION OF ACTIVE, SECRETED HUMAN PROSTATE-SPECIFIC ANTIGEN BY RECOMBINANT BACULOVIRUS-INFECTED INSECT CELLS ON A PILOT-SCALE" BIO/TECHNOLOGY, vol. 13, no. 11, November 1995, pages 1230-1234, XP000604552
- KARR J F ET AL: "THE PRESENCE OF PROSTATE-SPECIFIC ANTIGEN-RELATED GENES IN PRIMATES AND THE EXPRESSION OF RECOMBINANT HUMAN PROSTATE-SPECIFIC ANTIGEN IN A TRANSFECTED MURINE CELL LINE" CANCER RESEARCH, vol. 55, no. 11, 1 June 1995, pages 2455-2462, XP000605373 cited in the application
- LOEVGREN J ET AL: "PRODUCTION OF RECOMBINANT PSA AND HK2 AND ANALYSIS OF THEIR IMMUNOLOGIC CROSS-REACTIVITY" BIOCHEMICAL AND BIOPHYSICAL RESEARCH COMMUNICATIONS, vol. 213, no. 3, 24 August 1995, pages 888-895, XP000605371
- HILL T ET AL: "Development of PROhK2 specific assay." 24TH MEETING OF THE INTERNATIONAL SOCIETY FOR ONCODEVELOPMENTAL BIOLOGY AND MEDICINE ON THE INTERDEPENDENCE OF TUMOR BIOLOGY AND CLINICAL ONCOLOGY, SAN DIEGO, CALIFORNIA, USA, NOVEMBER 17-22, 1996. TUMOR BIOLOGY 18 (SUPPL. 1). 1997. 7. ISSN: 1010-428, XP002075016
- DATABASE MEDLINE US NATIONAL LIBRARY OF MEDICINE (NLM), BETHESDA, MD, US PETTERSSON K ET AL: "Free and complexed prostate-specific antigen (PSA): in vitro stability, epitope map, and development of immunofluorometric assays for specific and sensitive detection of free PSA and PSA-alpha 1-antichymotrypsin complex [see comments]." XP002074992 & CLINICAL CHEMISTRY, (1995 OCT) 41 (10) 1480-8. JOURNAL CODE: DBZ. ISSN: 0009-9147., United States
- KUMAR A ET AL: "Expression of pro form of prostate-specific antigen by mammalian cells and its conversion to mature, active form by human kallikrein 2." CANCER RESEARCH, (1997 AUG 1) 57 (15) 3111-4. JOURNAL CODE: CNF. ISSN: 0008-5472., XP002074989 United States
- TAKAYAMA T K ET AL: "Characterization of the precursor of prostate-specific antigen. Activation by trypsin and by human glandular kallikrein." JOURNAL OF BIOLOGICAL CHEMISTRY, (1997 AUG 22) 272 (34) 21582-8. JOURNAL CODE: HIV. ISSN: 0021-9258., XP002074990 United States
- LOVGREN J ET AL: "Activation of the zymogen form of prostate-specific antigen by human glandular kallikrein 2." BIOCHEMICAL AND BIOPHYSICAL RESEARCH COMMUNICATIONS, (1997 SEP 18) 238 (2) 549-55. JOURNAL CODE: 9Y8. ISSN: 0006-291X., XP002074991 United States

## Description

### Field of the Intention

The present invention relates generally to the detection and identification of proteins, as well as various forms and subunits of proteins, which have potential utility as diagnostic markers. In particular, the present invention relates to the detection of inactive precursor forms of prostate specific antigen.

### Background of the Invention

Prostate cancer (PCa) is the most frequently diagnosed cancer in American males. Parker, S.L. et al., CA Cancer J. Clin., 46:5-27, 1996. Prostate specific antigen, or PSA, has been widely used as a reliable prognostic marker in the management of patients with prostate cancer. Catalona, W.J. et al., N. Engl. J. Med., 324:1156-1161, 1991; Oesterling, J.E., J. Urol., 145:907-923, 1991; Labrie, F. et al., J. Urol., 147:846-852, 1992. One of the major limitations of the PSA test is its lack of specificity to distinguish between benign prostatic hyperplasia (BPH) and PCa. McCormack, R.T. et al., Urology, 45:729-744, 1995. To improve the diagnostic accuracy of serum PSA, different approaches, such as PSA density, PSA velocity, ratio between free and total PSA or ratio between complex and total PSA have been introduced. Benson, M.C. et al., J. Urol., 147:815-816, 1992; Carter, H.B. et al., J. Am. Med. Assoc., 267:2215-2220, 1992; Oesterling, J.E. et al., J. Am. Med. Assoc., 270:860-864, 1993.

PSA (also known as hK3), a member of human kallikrein family of serine proteases, is a 30-34 kDa single chain glycoprotein with one N-linked oligosaccharide attached to asparagine 45. Belanger, A. et al., Prostate 27:187-197 1995. Molecular cloning of cDNA reveals that the mRNA of PSA codes for a 261 amino acid (aa) preproprotein in which a hydrophilic signal sequence of 17 aa (prepro region) and a propeptide of 7 aa precedes the mature protein of 237 aa. Lundwall, A. et al., FEBS Lett., 214:317-322, 1987; Riegman, P.H.J. et al., Biochem. Biophys. Res. Commun., 155:181-188, 1988; Henttu, P. et al., Biochem. Biophys. Res. Commun., 160:903-910,1989. PSA mRNA expression is predominant in prostate epithelium, Qui, S. et al., J. Urol. 144:1550-1556, 1990, and is regulated by androgens, Young, C.Y.F. et al., Cancer Res., 51:3748-3752, 1991. PSA has been shown to have chymotrypsin-like activity. Watt, K.W.K. et al., Proc. Natl. Acad. Sci. USA, 83:3166-3170, 1986; Ban, Y. et al., Biochem. Biophys. Res. Commun., 123:482-488, 1984. The proposed primary biological role of PSA is to cleave the major gel forming proteins, semenogelin I, II and fibronectin in seminal fluid, resulting in enhanced sperm motility. Lilja, H., J. Clin. Invest., 76:1899-1903, 1985. More recently, PSA has been reported to cleave the IGF binding protein 3, resulting in increased IGF activity, a function that may be important in regulation of epithelial cell growth in the prostate. Kanety, H. et al., J. Clin. Endocrin. Metab., 77:229-233, 1992. Although the aforementioned studies emphasize the possible physiological substrates of PSA, the fundamental questions regarding its biosynthesis, activation and regulation of activity remain unanswered.

The human kallikrein family consists of three members designated as hK1, hK2 and hK3 (PSA). Clements, J.A., Endocr. Rev., 10:393-419, 1989; Carbini, L.A. et al., J. Hypertens., 11:893-898, 1993. hK1 is primarily produced in the kidney, pancreas and submandibular salivary gland. Fukushima, D. et al., Biochemistry, 24:8037-8043, 1985. hK2, like PSA. is produced predominantly in the prostate epithelium (Morris, B.J., Clin. Exp. Pharm. Phys., 16:345-351, 1989; Chapdelaine, P. et al., FEBS Lett., 236:205-208, 1988), is regulated by androgens (Young, C.Y.F. et al., Biochemistry, 31:818-824, 1992; Grauer, L. et al, J. Androl., 17:353-359, 1996) and shares 78% aa homology with PSA (Schedlich, L.J. et al., DNA, 6:429-437, 1987; Lilja, H., World J. Urol., 11: 188-191, 1993). The attributes of hK2 as a potential prostate cancer marker have recently been reviewed. Young. C.Y.F. et al., The Prostate Supplement, 7:17-24, 1996; Darson, M.F. et at., Human glandular kallikrein 2 (hK2) expression in prostate intraepithelial neoplasia and adenocarcinoma: A novel prostate cancer marker, Urology 49:857-862, 1997; Rittenhouse, H.G. et al., Characterization and evaluation of hK2: a potential prostate cancer marker, closely related to PSA, Proceedings of the First International Consultation on Prostate Cancer, Monaco, 1996, 25p., 1997. PSA is a chymotrypsin-like protease whereas hK2 is a trypsin-like protease, Mikolajczyk, S.D. et al., Human glandular kallikrein (hK2) shows arginine-restricted specificity and forms complexes with plasma protease inhibitors, Prostate 34:44-50, 1998 indicating that the two enzymes have different physiological roles. Little is known about the physiological role(s) of hK2. Deperthes et al. have recently shown that fibronectin present in ejaculate was hydrolyzed more efficiently by hK2 than by PSA (J. Androl., 17: 659-665, 1996). On the other hand, semenogelins were hydrolyzed by hK2 to a similar extent as that of PSA. While PSA and hK2 have been shown to be present in the same environment, their interaction with each other has not been studied.

To study biosynthesis, regulation of activation of PSA and to explore the physiological relationship between hK2 and PSA, it is imperative to express PSA in mammalian cells. Several attempts have been made to express both PSA and hK2 in mammalian cells. The expression and purification of hK2 in mammalian cells has previously been reported. Kumar, A. et al., Cancer Res., 56:5397-5402, 1996. Lovgren et al. expressed PSA in BHK21 cells using the SFV system (Lovgren, J. et al., Biochem. Biophys. Res. Commun., 231:888-895, 1995). This system, however, did not provide a stable cell line necessary for studying the biosynthetic processing of PSA. Although Karr et al. generated a stable murine colon adenocarcinoma cell line expressing PSA with an objective to use them as targets for anti-PSA therapies, biochemical studies on PSA were not reported. Karr, et al.,Cancer Res., 55:2455-2462, 1995.

Thus, there is a need for a method to stably express PSA in a mammalian system. There is also a need to detect expression products of such a system, including PSA precursors and derivatives. Further, there is a need to improve the diagnosis and staging of prostate cancer and to better distinguish benign prostatic hyperplasia from prostate cancer.

### Summary of the Invention

An expression vector is described herein which clones and stably expresses PSA in mammalian cells. It is herein demonstrated for the first time that PSA is secreted into the spent media by mammalian cells as proPSA. The proPSA thus secreted is enzymatically inactive and stable in the media. It is also demonstrated that proPSA can be converted to enzymatically active PSA in the extracellular medium by hK2. This suggests a possible physiological relationship between hK2 and PSA and that hK2 could be regulating PSA activity in vivo.

Further herein described is a chimeric expression vector comprising a nucleic acid molecule. The nucleic acid molecule encodes a pPSA polypeptide. The nucleic acid molecule is preferably operably linked to control sequences which are recognized by a host cell that is transformed with the expression vector. The host cell is preferably derived from a mammalian source.

Whereas, the present invention provides a method for detecting proPSA in a human physiological fluid, this aspect of the invention is based on the discovery that proPSA exists in biological fluid and may serve as a useful marker for prostate cancer. Specifically, several inactive precursor forms of PSA have been identified and at least one has been detected in serum. The measurement of these inactive precursor forms of PSA may provide important information regarding the diagnosis and staging of prostate cancer.

Therefore, proPSA polypeptides, as well as variants and subunits thereof, produced by the methods of the present invention can be used to produce populations of antibodies that, in turn, can be used as the basis for direct or competitive assays to detect and quantify proPSA polypeptides (or "protein") in samples derived from physiological fluids, such as seminal fluid, blood or serum, tissues, such as prostate carcinomas, or cells, such as prostate cells.

Another embodiment of the present invention, therefore, provides an antibody, and preferably a monoclonal antibody, which specifically binds to proPSA. Such antibodies specifically bind to the various inactive precursor forms of proPSA, including [-1]pPSA, [-2]pPSA, [-[-3]pPSA, [-4]pPSA, [-5]pPSA, [-6]pPSA and [-7]pPSA. Hybridoma cell lines producing such antibodies are also provided by way of the present invention.

Direct and competitive assays to detect proPSA are also included within the scope of the present invention. A method for detecting proPSA in a sample of human physiological fluid is described which includes providing purified antibodies to pPSA, contacting the antibodies with the sample to allow formation of complexes between the antibodies and pPSA, and determining the presence or amount of pPSA complexed with the antibodies. Diagnostic methods and kits are also included as embodiments of the present invention.

### Brief Description of the Drawings

FIG. 1 is a schematic representation of PSA expression vector, pGTD-PSA.
FIG. 2 depicts the expression ofPSA by AV12-PSA#8 cells. Serum containing spent media of AV12-PSA#8 cells was harvested each day for 6 consecutive days. PSA concentration was measured using Tandem^{®}-MP PSA assay. Viable cells were counted each day using trypan blue.

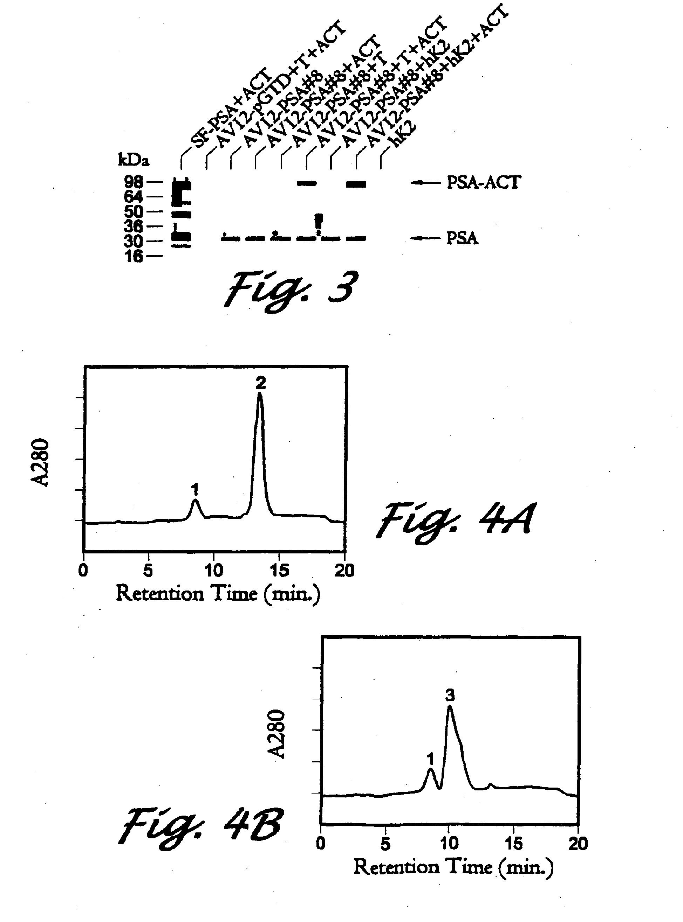

FIG. 3 shows trypsin and hK2 treated AV12-PSA#8 spent media formed a complex with ACT. The day 7 spent media of AV12-PSA#8 cells and AV12-PGTD cells were appropriately concentrated. Concentrated spent media (containing 1 µg of PSA measured by the Tandem^{®}-MP PSA assay) was mixed with purified trypsin (0.02 µg) or purified hK2 (1 µg) for 60 minutes at 37°C. The reactions were quenched by adding 0.05 µg of aprotinin. Some of the samples were incubated with ACT (5 µg) for an additional 4 hours. The reactions were stopped with sample buffer+βME and boiling. Approximately 1/10 of the reaction volume was electrophoresed on a 4-20% gradient gel. Proteins were electroblotted on nitrocellulose filter and probed with PSA-specific mAb PSM 773. Molecular weight markers are indicated on the left hand side. SF-PSA=PSA purified from seminal fluid (SF), AV12-PSA#8=Spent media from AV12-PSA#8 cells, T=Trypsin. ACT=α1-antichymotrypsin.
FIG. 4 depicts hydrophobic interaction chromatography (HIC) of hK2 incubated with purified proPSA. Panel A: hK2 mixed with pPSA and injected immediately. Peak 1 is hK2 and peak 2 is purified pPSA. Panel B: hK2 incubated with pPSA for 2 hours at 37°C. Peak 3 is mature PSA as confirmed by N-terminal sequencing.
FIG. 5 depicts the HIC chromatographic profile of PSA and ACT standards. FIG. 5A shows the retention time for active ACT. FIG. 5B shows active ACT incubated with PSA which was purified from seminal fluid. FIG. 5C shows the retention time for seminal fluid PSA.
FIG. 6 depicts the HIC chromatographic profile of the different forms of PSA. FIG. 6A shows the retention time for mature PSA and pPSA, including [-4]pPSA, [-5]pPSA and [-7]pPSA. FIG. 6B shows the retention time for PSA forms from a serum sample bound to a PSM773 affinity column.
FIG. 7 depicts the chromatographic profile for a mixture of purified mature PSA and pPSA. FIG. 7B shows the chromatographic profile for the protein mixture without the addition of ACT. FIG. 7A shows the chromatographic profile for the same protein mixture after incubation with ACT for 2 hours at 37°C.
FIG. 8 depicts Western blotsin which a 33 kDa form of PSA in prostate carcinoma serum is identified.
FIG. 9 depicts Western blot analysis of PSA in serum and seminal fluid. Lane 1: prostate carcinoma sera (1 µl, 28 ng PSA loaded per lane); Lane 2: 28 ng PSA purified from seminal fluid; Lane 3: 28 ng PSA purified from seminal fluid spiked into 1 µl of female serum; Lane 4: molecular weight markers.

### Detailed Description of the Preferred Embodiments

The identification of inactive precursor forms of PSA in serum suggests that measuring serum concentrations of proPSA can be useful in the diagnosing and monitoring of prostate cancer. In order to discern the steps involved in biosynthesis of PSA and the activation of proPSA to mature PSA, expression of PSA in mammalian cells is necessary.

As used herein, the terms "PSA" and "PSA polypeptide" are used interchangeably and include recombinant prepro, pro and mature PSA polypeptides. The terms "proPSA", "pPSA", "proPSA polypeptide" and "pPSA polypeptide" are used interchangeably and preferably encompass all inactive precursor forms of PSA, but particularly [-4]proPSA, [-7]proPSA, [-5]proPSA, [-1]proPSA, [-2]proPSA, [-3]proPSA and [-6]proPSA.

As used herein, "chimeric" means that a vector comprises DNA from at least two different species, or comprises DNA from the same species, which is linked or associated in a manner which does not occur in the "native" or wild type of the species.

"Control sequences" is defined to mean DNA sequences necessary for the expression of an operably linked coding sequence in a particular host organism. The control sequences that are suitable for prokaryotic cells, for example, include a promoter, and optionally an operator sequence, and a ribosome binding site. Eukaryotic cells are known to utilize promoters, polyadenylation signals and enhancers.

"Operably linked" means that the nucleic acids are placed in a functional relationship with another nucleic acid sequence. For example, DNA for a presequence or secretory leader is operably linked to DNA for a polypeptide if it is expressed as a preprotein that participates in the secretion of the polypeptide; a promoter or enhancer is operably linked to a coding sequence if it affects the transcription of the sequence; or a ribosome binding site is operably linked to a coding sequence if it is positioned so as to facilitate translation. Generally, "operably linked" means that the DNA sequences being linked are contiguous and, in the case of a secretory leader, contiguous and in reading phase. However, enhancers do not have to be contiguous. Linking is accomplished by ligation at convenient restriction sites. If such sites do not exist, the synthetic oligonucleotide adaptors or linkers are used in accord with conventional practice.

The general methods for constructing recombinant DNA which can transform target cells are well known to those skilled in the art, and the same compositions and methods of construction may be utilized to produce the DNA useful herein. For example, J. Sambrook et al., Molecular Cloning: A Laboratory Manual, Cold Spring Harbor Press (2d ed., 1989), provides suitable methods of construction.

The recombinant DNA can be readily introduced into the target cells by transfection with an expression vector comprising cDNA encoding PSA, for example, by the modified calcium phosphate precipitation procedure ofC. Chen et al., Mol. Cell. Biol., 7, 2745 (1987). Transfection can also be accomplished by lipofection, using commercially available kits, e.g., provided by Life Technologies, Inc. (Gibco BRL), Rockville, MD USA.

Suitable host cells for the expression of PSA are derived from multicellular organisms. Such host cells are capable of complex processing and glycosylation activities. However, mammalian cells are the preferred host for expression of mammalian protein since these cells modify and process the recombinant protein in a manner closely related to the natural host of the protein. In principle, any higher eukaryotic cell culture can be employed in the practice of the invention, whether from vertebrate or invertebrate culture. Examples of invertebrate cells include plant and insect cells. Numerous baculoviral strains and variants and corresponding permissive insect host cells have been identified.

"Polymerase chain reaction" or "PCR" refers to a procedure or technique in which amounts of a preselected fragment of nucleic acid, RNA and/or DNA, are amplified as described in U.S. Patent No. 4,683,195. Generally, sequence information from the ends of the region of interest or beyond is employed to design oligonucleotide primers. These primers will be identical or similar in sequence to opposite strands of the template to be amplified. PCR can be used to amplify specific RNA sequences, specific DNA sequences from total genomic DNA, and cDNA transcribed from total cellular RNA, bacteriophage or plasmid sequences, and the like. See generally Mullis et al., Cold Spring Harbor Symp. Quant. Biol., 51, 263 (1987); Erlich, ed., PCR Technology, (Stockton Press, NY, 1989).

When PSA polypeptide is expressed in a recombinant cell other than one of human origin, the PSA polypeptide is completely free of proteins or polypeptides of human origin. However, it is necessary to purify PSA polypeptide from recombinant cell proteins or polypeptides to obtain preparations that are substantially homogeneous as to PSA polypeptide. For example, the culture medium or lysate can be centrifuged to remove particulate cell debris. The membrane and soluble protein fractions are then separated. The PSA polypeptide may then be purified from the soluble protein fraction, and if necessary, from the membrane fraction of the culture lysate. PSA polypeptide can then be purified from contaminant soluble proteins and polypeptides by fractionation on immunoaffinity or ion exchange columns; ethanol precipitation; reverse phase HPLC; chromatography on silica or on an anion exchange resin such as DEAE; chromatofocusing; SDS-PAGE; ammonium sulfate precipitation; gel filtration using, for example, Sephadex G-75; or ligand affinity chromatography.

Once isolated, and in accordance with an embodiment of the present invention, a proPSA polypeptide or peptide corresponding to the proPSA region can be used to produce anti-pPSA antibodies. The proPSA polypeptides used to generate antibodies in accordance with the invention include, but are not limited to -7, -5 and -4 proPSA. Peptides corresponding to the proPSA region can also be used to generate anti-pPSA antibodies and include all peptides which contain any portion of the pro region of the pPSA polypeptide. These peptides preferably contain about 8 to 15 amino acids and comprise an immunogenic epitope. Monoclonal antibodies against purified pPSA (total protein) or the above peptides can be prepared using known hybridoma cell culture techniques, for example, as described by E. Harlow et al., Antibodies: A laboratory manual, Cold Spring Harbor Laboratory. 1988. In general, this method involves preparing an antibody-producing fused cell line, e.g., of primary spleen cells fused with a compatible continuous line of myeloma cells, and growing the fused cells either in mass culture or in an animal species from which the myeloma cell line used was derived or is compatible. Such antibodies offer many advantages in comparison to those produced by inoculation of animals, as they are highly specific and sensitive and relatively "pure" immunochemically. Immunologically active fragments of antibodies are also within the scope of the present invention, e.g., the F(ab) fragment, as are partially humanized monoclonal antibodies.

As well as being useful as an antigen to produce the present anti-pPSA antibodies, the isolated pPSA polypeptide produced in accordance with a method of the present invention and its antigenically active variants, derivatives and fragments thereof can be used in assays for proPSA in samples derived from biological materials suspected of containing proPSA or anti-proPSA antibodies.

A two-antibody sandwich technique can be used primarily to determine the antigen concentration in unknown samples. Two-antibody assays are quick and accurate, and if a source of pure antigen, in this case proPSA, is available, the assays can be used to determine the absolute amounts of antigen in unknown samples. The assay requires two antibodies that bind to non-overlapping epitopes on the antigen. Either two monoclonal antibodies that recognize discrete sites or one batch of affinity-purified polyclonal antibodies can be used.

In a two-antibody assay, one antibody is purified and bound to a solid phase. Any solid phase can be used, however, for most applications, a PVC microtiter plate is preferred. The antibody bound, for example to a well of a microtiter plate, is unlabelled and is referred to as the "capture antibody." The amount of antibody to be used will depend on the individual assay, but an amount of about 1 µg/well generally gives maximal binding. Higher or lower amounts of capture antibody can also be used. The wells can then be washed and sample added to the wells to allow the antigen, in this case pPSA, in the test solution to bind to the solid phase. Unbound proteins can be removed by washing, and a labeled second antibody can be added. Alternatively, the sample and the second labeled antibody can be added simultaneously. After washing, the assay can be quantitated by measuring the amount of labeled second antibody that is bound to the solid phase. A most preferred embodiment of the present invention utilizes a monoclonal antibody as the first unlabeled antibody and a monoclonal antibody as the second labeled antibody. The detection method used to quantitate the amount of bound labeled antibody depends on the label used. Antibodies can be labeled conveniently with iodine, enzymes or biotin. Calorimetric or other detection methods can be used.

The proPSA polypeptides of the present invention can be immobilized and used as "capture antigens" to bind and immobilize anti-pPSA antibodies from a sample to be assayed for anti-pPSA antibodies. The bivalent complex of proPSA polypeptides and anti-pPSA antibodies is then detected, e.g., in the case of human physiological material, by reacting it with an anti-human IgG antibody which comprises a detectable label or a binding site for a detectable label. In the latter case, the binding site is itself reacted with a compound specific for the binding site, which itself comprises a detectable label. Useful detectable labels include enzymes, radiolabels or fluorescent labels. The resultant ternary or quaternary complex can then be detected and/or quantified via the detectable label, i.e., via an enzyme-substrate color-forming reaction, radioemission, agglomeration, and the like.

Alternatively, the proPSA polypeptide can be labeled with a detectable label, such as via one or more radiolabeled peptidyl residues, and can be used to compete with endogenous proPSA for binding to anti-proPSA antibodies, i.e., as a capture antigen to bind to anti-proPSA antibodies in a sample of a physiological fluid, via various competitive immunoassay formats. For example, a competitive immunoassay for detecting or determining proPSA in a sample of human physiological fluid containing pPSA comprises:
(a) providing an amount of purified antibodies which react with pPSA selected from [-1]pPSA, [-2]pPSA, [-3]pPSA [-4]pPSA, [-5]pPSA, [-6]pPSA and [-7]pPSA and which do not display significant reactivity to the mature form of PSA, wherein the antibodies are raised against a recombinant pPSA expressed by a mammalian cell;
(b) mixing the sample to be tested with a known amount of a proPSA polypeptide or an immunoreactive subunit thereof that binds to an antibody which binds to said pPSA which comprises a detectable label, to produce a mixed sample;
(c) contacting said antibodies with said mixed sample for a sufficient time to allow immunological reactions to occur between said antibody and said proPSA in said sample and between said antibody and said labeled proPSA polypeptide;
(d) separating the antibody from the mixed sample;
(e) detecting or determining the presence or amount of labeled polypeptide either bound to the antibody or remaining in the mixed sample; and
(f) determining from the result in step (e) the presence or amount of said proPSA in said sample.

The immunoassays as described in detail above can be used in a method to detect pPSA in human physiological samples, such as serum and tissue, for the purpose of diagnosing and monitoring prostate cancer. The assays of the present invention can also be used in a method to distinguish prostate cancer from benign prostatic hyperplasia in which the method includes determining the amount of [-1]pPSA, [-2]pPSA, [-3]pPSA [-4]pPSA. [-5]pPSA. [-6]pPSA and [-7]pPSA in a sample, wherein said determination does not use antibodies that display significant binding to the mature form of PSA. The ratio of the amount of proPSA to total PSA in a sample could also be determined by the methods described herein.

The invention is defined by the claims appended hereto and is further described by reference to the following detailed examples.

### EXAMPLE 1

Expression of pPSA and Conversion to PSA by hK2

### Materials and Methods:

### Expression vector, cell line and transfection

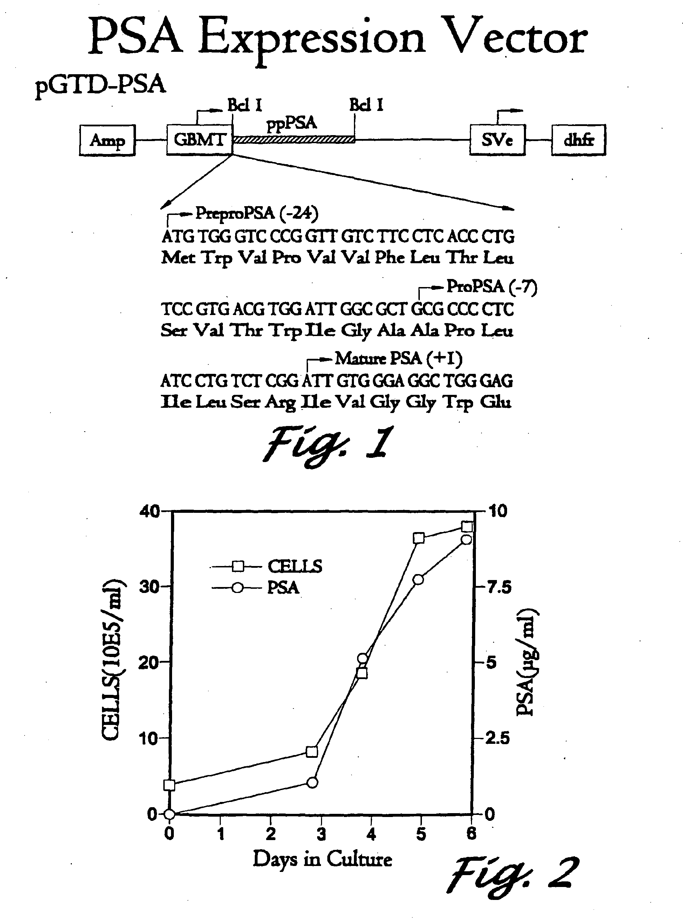

A 0.8-kb DNA fragment coding for entire ppPSA was cloned into the Bcl1 site of pGT-d under the control of GBMT promoter (Berg. D.T. et al., Ela-responsive mammalian host/vector system for the stable high-level expression of secreted proteins, Nucl. Acids Res., 20:5485-5486, 1992) resulting in the expression vector pGTD-PSA (Figure 1). The orientation and the sequence of the insert was confirmed. AV12-664 (ATCC CRL 9595), cultured in DMEM (high glucose) and 10% fetal clone (HyClone Logan, UT USA), were transfected with pGTD-PSA using Lipofectamine (Life Technologies, Inc (Gibco BRL), Rockville, MD, USA.). Transfected. AV12-664 cells (AV12-PSA) were selected in 400 nM methotrexate (Sigma Chemical Company St Louis, MO, USA). AV 12-664 transfected with the empty vector (AV 12-PGTD) were also selected in a similar manner for use as negative control. Single cell clones were isolated. Viability of cells was assessed by trypan blue dye exclusion.

### Antibodies

Murine mAbs PSM 773 and HKIG 586.1 were used. PSM 773 is one of the components of Tandem®-MP PSA kit (Hybritech Incorporated, San Diego, CA, USA) and has been shown to be specific for PSA (Wang, T.J. et al., Analysis of cross-reactivity of anti-PSA monoclonal antibodies with recombinant human glandular kallikrein, J. Urol., 155:695A, 1996). HK1G 586.1 is an anti-hK2 antibody. Kumar A. et al., Cancer Research, 56:5397-5402, 1996.

### ELISA and Western Blot analyse

Serum containing spent media from AV12-PSA cells was collected on specified days. PSA was measured using Tandem®-MP PSA and *free* PSA assays (both from Hybritech Incorporated, San Diego, CA, USA) according to manufacturer's instructions. Spent media from AV12 and AV 12-PGTD cells were used as negative controls.

For Western blot analysis, tissue culture flasks containing AV12-PSA cells were grown to approximately 60-70% confluency, washed with PBS and serum free HH4 media was added. Spent media harvested on specified days was concentrated using a Centricon 10 (Amicon, Inc, Beverly, MA, USA), incubated with either purified trypsin or purified hK2 followed by ACT and subjected to SDS-PAGE on a 4-20% gel (Bio-Rad, Inc, Hercules, CA, USA). After electrophoresis, proteins were electroblotted onto nitrocellulose membranes. Primary antibodies (1-10 µg/ml) and secondary antibodies (goat anti-mouse IgG-horseradish peroxidase, 1:500; Jackson Immunosearch Laboratories, Inc, West Grove, PA, USA) were used to probe the blots. The immunoreactive signals were detected using the enhanced chemiluminescence (Amersham, Buckinghamshire, England, United Kingdom) system according to the manufacturer's instructions.

### Purification of PSA

mAb PSM 773 was coupled to AminoLink (Pierce Biotechnology Rockford. IL, USA) according to the manufacturer's instructions. Tissue culture flasks containing AV 12-PSA cells were grown to approximately 60-70% confluency, washed with PBS and serum free HH4 media was added. Spent media was harvested on specified days, concentrated and incubated with the above resin overnight at 4°C with stirring. The resin was then made into a column, washed with PBS and the PSA was eluted with 100 mM glycine, 0.5 M NaCl, pH 2.5. The samples were immediately neutralized with 1 M TRIS, pH 8.0.

### Assay for the measurement of PSA activity

Enzymatic activity of PSA was measured according to the procedure published by Christensson, A. et al., Enzymatic activity of prostate-specific antigen and its reactions with extracellular serine proteinase inhibitors, Eur. J. Biochem., 194:755-763, 1990. Briefly, PSA preparations (either purified from seminal fluid or day 7 spent media of AV 12-PSA#8 cells) were incubated with 1 mM pNA-derivatized peptide chromogenic substrates (methoxysuccinyl-Arg-Pro-Tyr-pNA, S2586; Pharmacia Hepar, Inc, Franklin, OH, USA) in 200 mM TRIS 5 mM EDTA (pH 8.0) at 37°C. The enzymatic activity of PSA was determined by hydrolysis of the peptide chromogenic substrates, leading to an increase in absorbance at 405 nm.

### Conversion of purified proPSA to PSA by purified hK2

Purified pPSA (4.7 µM) was incubated with purified hK2 (0.56 µM) at 37°C in 100 mM TRIS 2 mM EDTA, pH 8. Affinity purification of hK2 is described by Kumar, A. et al., Expression of human glandular kallikrein, hK2, in mammalian cells, Cancer Res. 56:5397-5402, 1996 and Mikolajczyk, S.D. et al., Human glandular kallikrein (hK2) shows arginine-restricted specificity and forms complexes with plasma protease inhibitors, Prostate 34:44-50, 1998. The initial mixing was performed at 4°C and an aliquot was immediately resolved by hydrophobic interaction chromatography (HIC). The sample was placed at 37°C and additional aliquots were analyzed at times up to 2 hours. HIC column specifications and buffer compositions were as follows: polypropylaspartamide column, 4.6 X 250 mm, PolyLC (distributed by Western Analytical, Temecula, CA, USA), Buffer A: 1.2 M sodium sulfate, 50 mM sodium phosphate, pH 6.3; Buffer B: 50 mM sodium phosphate, 5% v/v 2-propanol, pH 7.3. Samples were prepared in 1.5 M ammonium sulfate then injected on the HIC column with the following gradient: 0-35% Buffer B from 0 to I minute; 30-80 % Buffer B from 1-12 minutes and isocratic at 80% buffer B from 12-14 minutes before re-equilibration in Buffer A. Peaks collected from the column were adsorbed to PVDF (polyvinylidene difluoride) membrane by using a prosorb cartridge (Applied Biosystems, Foster City, CA, USA) and subjected to N-terminal sequencing using an AB1 Model 492 Procise sequencer. The enzymatic activity of peak samples was determined as described above.

### Results:

The cDNA for PSA was cloned into the pGT-d vector under the control of the GBMT promoter using an approach similar to the one described for hK2 by Kumar A. et al., Cancer Res. 56:5397-5402, 1996. To study the expression of PSA, AV12 cells were transfected with the pGTD-PSA expression vector. Cells were selected in 400 nM methotrexate for 2-3 weeks, and single cell clones were analyzed for PSA expression using Tandem®-MP PSA assay and on Western blots using mAb PSM 773. Clone AV12-PSA#8 was selected based on its high expression levels of a PSA-immunoreactive band at ~32 kDa.

To determine the PSA expression pattern in mammalian cells, samples of spent media from AV12-PSA#8 cells were collected for 6 consecutive days and analyzed using the Tandem^{®}-MP PSA assay. Figure 2 shows that PSA was detected in spent media at day 1 and accumulated to >9µg/ml by day 6. Expression of PSA was higher during the log phase of cell growth, indicating that a stable form of PSA is secreted by the cells as opposed to being released following cell death and lysis. When the same samples were analyzed for free PSA, similar values were obtained (data not shown) indicating that AV12-PSA#8 cells express uncomplexed or free PSA.

To determine the identity of the protein that is secreted on day 1, the spent media from AV12-PSA#8 cells was collected and concentrated. The PSA in the media was purified by affinity chromatography using PSM 773, a PSA-specific mAb. N-terminal sequencing analysis of the purified protein revealed the sequence: APLILSRIVGG. This sequence corresponds with the sequence predicted for the terminus of proPSA that starts with -7 aa. Another species starting at -5 aa (leu) was also identified. No competing sequence corresponding to the mature form of PSA was evident from the profile of aa released sequentially by the Edman degradation procedure. When the same purification protocol was used to purify the protein from day 7 spent media of AV12-PSA#8 cells, only proPSA and not mature PSA was detected. This result indicates that PSA is secreted as proPSA by AV12-PSA#8 cells beginning from day 1 and proPSA is stable in the spent media even after 7 days.

To demonstrate that proPSA expressed by AV12-PSA#8 is enzymatically inactive and can be converted to enzymatically active PSA by mild trypsin treatment, a spectrophotometric assay was employed using the commercially available chromogenic substrate, methoxysuccinyl-Arg-Pro-Tyr-pNA (S-2586). A ten fold increase in activity (from 2.38x10-4 to 2.27x10-3 Activity Units/minute) was observed when spent media from AV12-PSA#8 (containing equivalent to 14 µg of PSA as measured by Tandem^{®}-MP PSA assay) was treated with trypsin (2% w/w) for 60 minutes at 37°C and quenched with 4% w/w aprotinin. Spent media from AV12-PGTD cells (AV12 cells transfected by empty pGT-d vector) that was similarly treated with trypsin and quenched with aprotinin, showed no detectable activity indicating that no endogenous PSA-like proteases are secreted by AV12 cells. The positive control, PSA (14 µg) purified from seminal plasma, and similarly treated with trypsin in HH4 exhibited 9.49x10-3 Activity Units/minute activity.

It has previously been shown that PSA complexes with ACT in serum. We inferred that enzymatically inactive proPSA would not complex with protease inhibitors such as ACT. To test whether the trypsin converted PSA would complex with ACT, ACT was added to the spent media of AV12-PSA#8 cells following preincubation with trypsin. After four hours, the reaction was stopped by addition of sample buffer+βME and boiling. The samples were then electrophoresed, electroblotted and probed with PSM 773 mAb (Figure 3). A -94 kDa complex comigrating with PSA-ACT (lane 1) was detected in trypsin treated spent media of AV 12-PSA#8 cells (lane 6). In contrast, PSA-ACT complex was not observed when untreated AV 12-PSA#8 spent media was incubated with ACT (lane 4). No immunoreactive band was observed when trypsin preincubated AV 12-PGTD day 7 spent media was incubated with ACT (lane 2). These results confirmed that proPSA in the day 7 spent media of AV 12-PSA#8 cells was enzymatically inactive and did not complex with the protease inhibitors, ACT. In addition, these results indicated that proPSA was converted to PSA by mild trypsin treatment and this PSA was enzymatically active and could covalently complex with ACT. It appears that -1 to +1 region of proPSA is the major site for trypsin reactivity as no PSA-degradation products were detected by Western blot using PF1D 215 mAb (data not shown). PF1D 215 mAb has previously been shown to detect the PSA degradation products present in seminal fluid. Wang, T.J. et al, Antibody Specificities for PSA and PSA Fragments by SDS-PAGE Western Blot Analysis, Tumor Biology 20:75-78, 1997.

hK2 is another member of human kallikrein family. It is predominantly expressed in the prostate epithelium, the site where PSA is also abundantly expressed. hK2 exhibits arginine restricted trypsin-like activity. To test whether proPSA can be converted to PSA by hK2, purified hK2 was added to the spent media of AV12-PSA#8 cells. After 60 minutes of incubation at 37°C, ACT was added to these samples. After another 4 hours of incubation at 37°C, the reaction was stopped by addition of sample buffer+βME and boiling. The samples were then electrophoresed, electroblotted and probed with PSM773 (Figure 3). A ~94 kDa band comigrating with purified PSA-ACT (lane 1) was detected in hK2 treated spent media of AV12-PSA#8 (lane 8). Although hK2 and PSA share ~78% aa similarity, hK2 was not immunodetected by PSM 773 mAb (lane 9) confirming the PSA specificity of this mAb. These results indicate that in AV 12-PSA#8 spent media hK2 can convert enzymatically inactive proPSA to an active form of PSA.

To confirm the above results, affinity purified preparations of hK2 and pPSA were incubated and the conversion of pPSA to PSA was analyzed by HIC and amino acid sequencing. The mixture was resolved over an HIC column as described above (Figure 4). Despite their similarities, hK2, pPSA and mature PSA have distinctly different retention times. Figure 4A shows the mixture of hK2 and pPSA at time 0 and Figure 4B shows the same mixture after a 2 hour incubation at 37°C. The retention times for hK2 (peak 1) and pPSA (peak 2) were identical to that of purified hK2 and pPSA, respectively when injected separately (data not shown). The peak of hK2-converted PSA (peak 3) eluted at the same position as mature PSA purified from seminal fluid (data not shown). Figure 4B shows that after an incubation of 2 hours at 37°C with purified hK2, the pPSA peak was almost entirely converted to mature PSA. Additional time points at 20 minutes and 1 hour were also analyzed and showed intermediate levels of PSA (data not shown). The conversion from the proPSA to mature PSA was confirmed by aa sequencing of the peaks which showed peak 2 to contain the N-terminal proPSA leader peptide (starting at -7 aa) while peak 3 began with the mature sequence (starting at +1 aa). Additionally, the hK2 converted PSA contained enzymatic activity of 58 nmoles/minute/mg on S-2586 substrate. This value was comparable to the enzymatic activity of PSA purified from seminal fluid on the same substrate. In contrast the pPSA sample prior to hK2 treatment contained <5% of the PSA enzymatic activity. Purified pPSA when incubated without hK2 showed no conversion to PSA (data not shown).

Like other serine proteases, PSA is translated as an inactive preproPSA precursor. After passage through the secretory pathway, the signal peptide is cleaved, yielding the pro form of the protein. Although the pro peptides are generally cleaved inside the cell (e.g., tissue plasminogen activator, protein C and tumor necrosis factor), there are exceptions (e.g., renin, trypsin, chymotrypsin and hK2) that are secreted as pro proteins and cleaved extracellularly (Kumar. A. et al., Cancer Res. 56: 5397-5402, 1996). The following evidence unequivocally demonstrates that PSA is secreted as proPSA from the mammalian cells: (a) spent media of AV 12-PSA#8 cells had no PSA-like enzymatic activity, (b) the PSA form in the spent media of AV12-PSA#8 cells did not complex with ACT as demonstrated by Western blot analysis, (c) trypsin treatment of spent media of AV 12-PSA#8 resulted in nature PSA that was enzymatically active and formed a complex with ACT, and (d) purification and sequence analysis of the PSA form present in the spent media of AV12-PSA#8 confirmed its identity as proPSA.

PSA exists in many forms in SF and in serum. These forms include complexes of PSA with protease inhibitors such as ACT and Protein C Inhibitor (PCI) PSA also exists as a PSA-MG PSA-alpha 2 macroglobulin complex and free PSA. It is possible that some of the free PSA in the serum and SF is in fact proPSA. Our data suggest that proPSA exists in biological fluids and may be a useful marker for prostatic diseases.

Thus far, the protease(s) responsible for cleaving the propeptide from proPSA to form the active molecule has not been identified. hK2, another member of human kallikrein family, exhibits arginine-restricted trypsin-like activity and similar to PSA, it is predominantly expressed in the prostate epithelium (Morris, B.J. *supra;* Chapdelaine, P., *supra*). hK2 is secreted as prohK2 by mammalian cells. prohK2 is converted to enzymatically active hK2 extracellularly. hK2 has been shown to possess autocatalytic activity as it can clip itself between residues -1-+1. Mikolajczyk, et al., Alanine 217 is important for the catalytic function and autoactivation of prostate-specific human kallikrein 2 . Eur. J. Biochem 246: 440-446, 1997.

The data presented herein clearly show that hK2 clips the propeptide from the proPSA converting it to enzymatically active mature PSA (Figure 4). Mature PSA but not proPSA could complex with ACT (Figure 3). This result suggests a possible physiological regulatory relationship between these two members of human kallikrein family. It is anticipated that further studies will determine if -1-+1 of PSA is the preferred site of action by hK2. Typically 30-40% PSA isolated from seminal fluid is clipped between residues 85-86, 145-146 or 182-183 (Christensson, A. et al., Eur. J. Biochem., 194:755-763, 199a). It is known that the clipping of PSA between 145-146 (lysine-lysine) inactivates PSA (Christensson, A. et al., *supra*). It is presumed that clipping between 85-86 and 182-183 also leads to inactivation. hK2 may be regulating PSA activity and complex formation with protease inhibitors by clipping at some of the above-mentioned sites. Similarly, PSA may also be regulating hK2 activity by clipping hK2 at chymotrypsin sensitive sites. The reagents described herein plus hK2 expressed in mammalian cells should facilitate these experiments.

The above results collectively indicate that PSA is expressed as the pro form in mammalian cells and can be converted to the enzymatically active mature form extracellularly by hK2. These results also suggest that proPSA may be present in biological fluids and therefore, could be a useful marker for prostatic diseases. The cell lines described herein constitute a valuable source of proPSA to be used as an immunogen and to study the biological roles of proPSA and PSA in greater detail.

### EXAMPLE 2

### Detection of pPSA in Human Serum

The presence of pPSA in human serum would indicate the following. First, that PSA is secreted as the pPSA form in human tissue and it is converted to mature PSA extracellularly. Second, that pPSA is stable in human serum and thus may be a useful diagnostic marker for prostate cancer (pCa) or BPH. We evaluated the presence of pPSA in human serum by first using affinity purification to purify all forms of PSA present in a pool of human serum. We next fractionated the eluted PSA forms on HPLC and identified each PSA form based on its elution profile from the column. This analysis indicated that pPSA is present in human serum.

### Methods:

75 mls of pooled human serum from prostate cancer patients with elevated PSA were obtained. Solid ammonium sulfate was added to the serum to make the final concentration 2 M, then the sample was dialyzed versus 2 M ammonium sulfate for 16 hours at 4°C. The serum was then clarified by centrifugation and the supernatant solution dialyzed 3 times (1 hour each time) against 2 liters of 20 mM sodium phosphate, pH 7. The sample was then filtered through a 0.2 µ membrane filter and passed over a 0.5 ml affinity column at 1 ml/min. The affinity column consisted of the mAb PSM773 covalently bound to Aminolink (Pierce Biotechnology Rockford, IL, USA) resin at a concentration of 5 mg mAbs per ml of resin.

The affinity column was washed with 50 mls of PBS and the PSA eluted with 3 X 1 ml volumes of 100 mM glycine, 0.5 M sodium chloride, pH 2.5. The eluant (3 mis) was neutralized with 300 µl of 1 M TRIS, pH 8. Ammonium sulfate was added to the eluant to a final concentration of 2 M and this sample was applied to an HPLC column to be resolved by hydrophobic interaction chromatography (PolyLC, polypropyl aspartamide column, 1000 A pore size, 4.6 mm X 200 mm distributed by Western Analytical, Temecula, CA USA). Buffer A was 20 mM sodium phosphate, 1.2 M sodium sulfate, pH 6.3 and Buffer B was 50 mM sodium phosphate, 5% 2-propanol, pH 7.4. The elution gradient was 0-35% B from 0-1 minute and 35-80% B from 1-14 minutes, before re-equilibration in Buffer A. The flow rate was 1 ml/minute.

### Results:

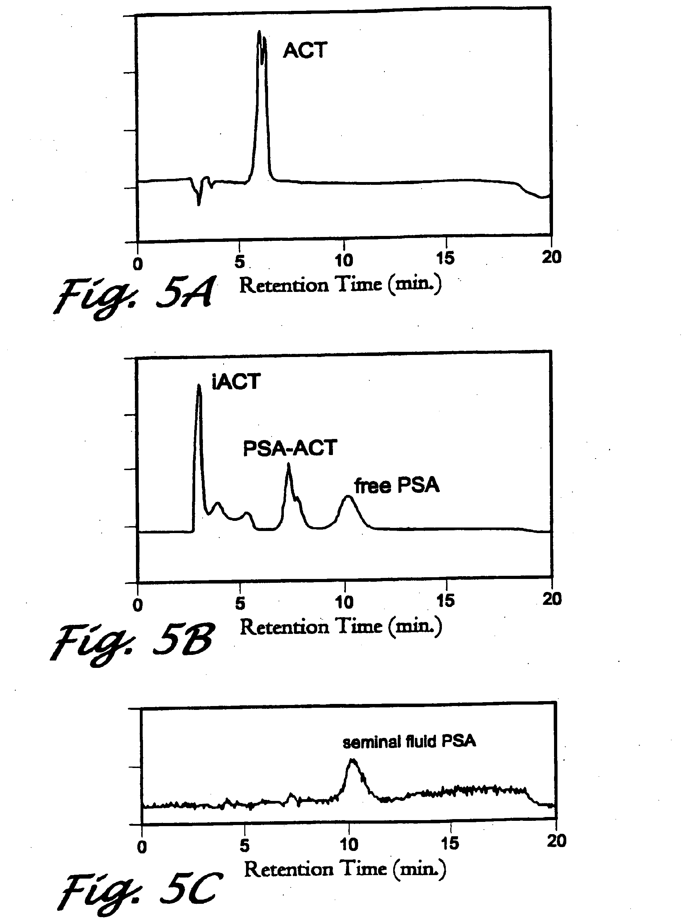

Figure 5 shows the retention times (RT) of standards resolved under the HIC chromatographic conditions described above. Figure 5A shows the RT of active ACT. Figure 5B shows the RT of active ACT incubated with PSA purified from seminal fluid. Because seminal fluid PSA is known to contain approximately 50% inactive PSA (known as "free" PSA because it will not complex with ACT), incubation of PSA with ACT results in PSA-ACT as well as free PSA. PSA also clips and inactivates ACT and so inactive ACT (iACT) is also seen. Figure 5C shows the RT of seminal fluid PSA. There is no difference between the retention times of active PSA and inactive PSA under these chromatographic conditions.

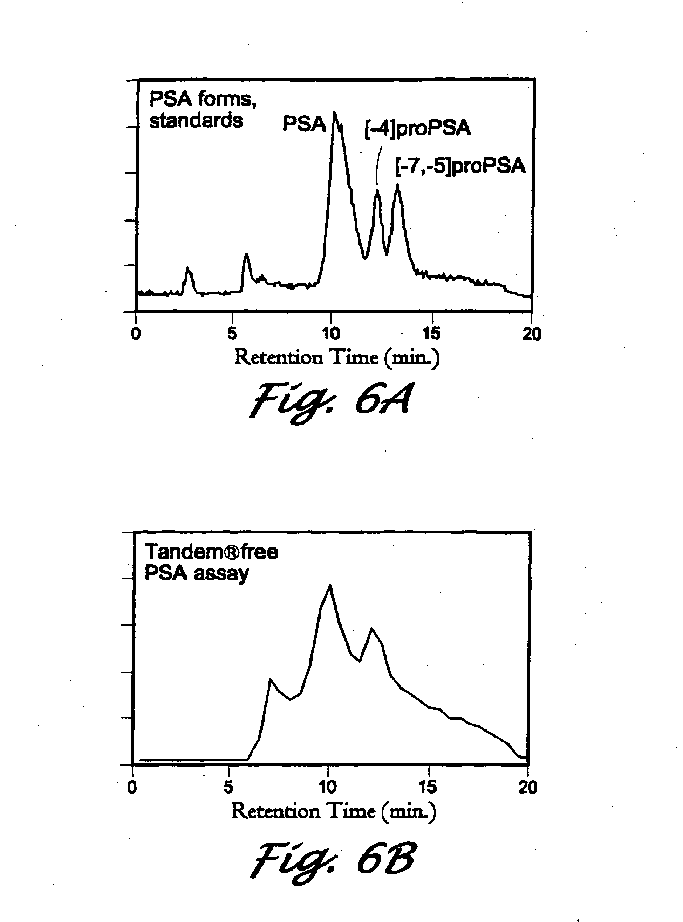

Figure 6A shows the RT of standards of the different forms of PSA. All forms were verified by amino acid sequencing. The [-7,-5]pPSA peak contains approximately equal levels of [-7]pPSA and [-5]pPSA forms which are not resolved from each other. Figure 6B shows the profile of PSA forms from serum bound to the PSM773 affinity column as described above. Samples were collected in 0.5 ml fractions and assayed by Tandem^{®}-MP *free* PSA assay (fPSA assay, Hybritech Incorporated, San Diego, CA, USA.). The fPSA assay detects both pPSA and free (inactive) PSA. The minor peak at 7 minutes is due to the slight cross-reactivity of the fPSA assay to the PSA-ACT eluted from the affinity column. The actual level of PSA-ACT in this sample is about 10 times higher than the level of free PSA (data not shown). The peaks at 10 minutes and 12 minutes correspond to mature PSA and [-4]pPSA, respectively. These data indicate that at least one form of pPSA ([-4]pPSA) is present in human serum and, as judged by the relative peak areas, it makes up approximately 25% of the free or uncomplexed PSA in serum.

### EXAMPLE 3

### Reactivity of Forms of PSA with ACT

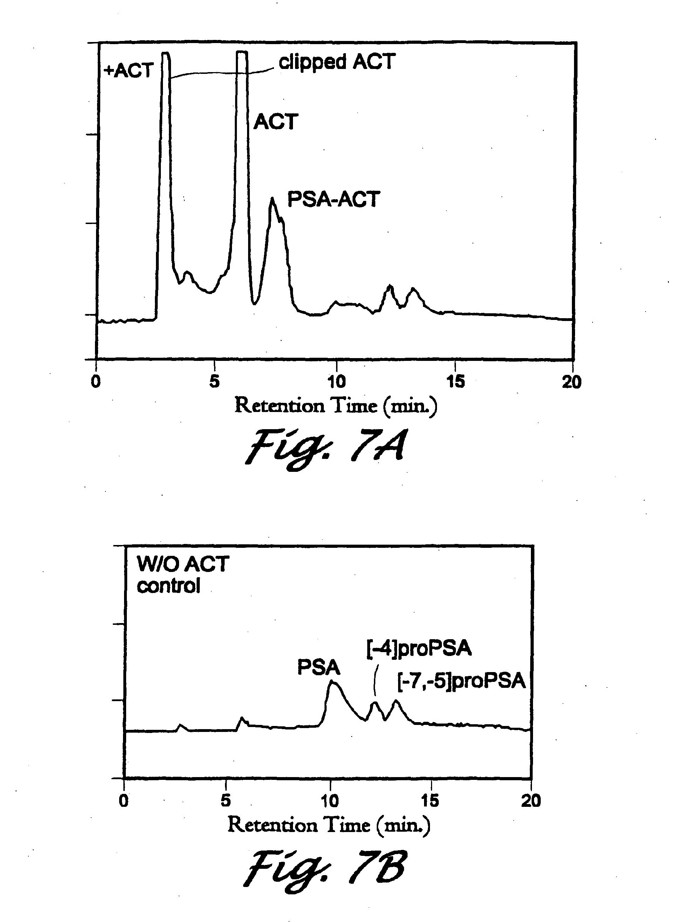

To confirm that the pro forms of PSA are not reactive with ACT, a mixture of purified mature PSA, [-4]pPSA and [-7,-5]pPSA were incubated with ACT. Figure 7B (W/O ACT) shows the chromatographic profile of the protein mixture without the addition of ACT. Figure 7A (+ACT) shows the chromatographic profile of an identical amount of the same mixture after incubation with ACT for 2 hours at 37°C. Only the mature PSA forms an ACT complex. The [-4], [-5], and [-7] forms of pPSA did not form a complex with ACT as they showed no decrease in peak area. This data is consistent with the [-4]pPSA in serum not being complexed with ACT.

### EXAMPLE 4

### Detection of Inactive Uncomplexed PSA in Human Sera via Western Blot

### Methods:

Three different specimens of prostate carcinoma serum (2 µl/lane) containing varying amounts of PSA (971 ng/ml - 5,330 ng/ml) were reduced and denatured and loaded on a 4%-20% polyacrylamide gel. The gel was blotted to nitrocellulose and incubated with the anti-PSA monoclonal antibody PSM773 at 2 µl/ml for I hour. Following extensive washing, secondary antibody conjugated to HRP was added and incubated with the blot for 1 hour and again washed with PBS. Antibody reactivity was detected using the enhanced chemiluminescence (ECL) reagent (Amersham, Arlington Heights, IL, USA.) according to manufacturer's instructions.

### Results:

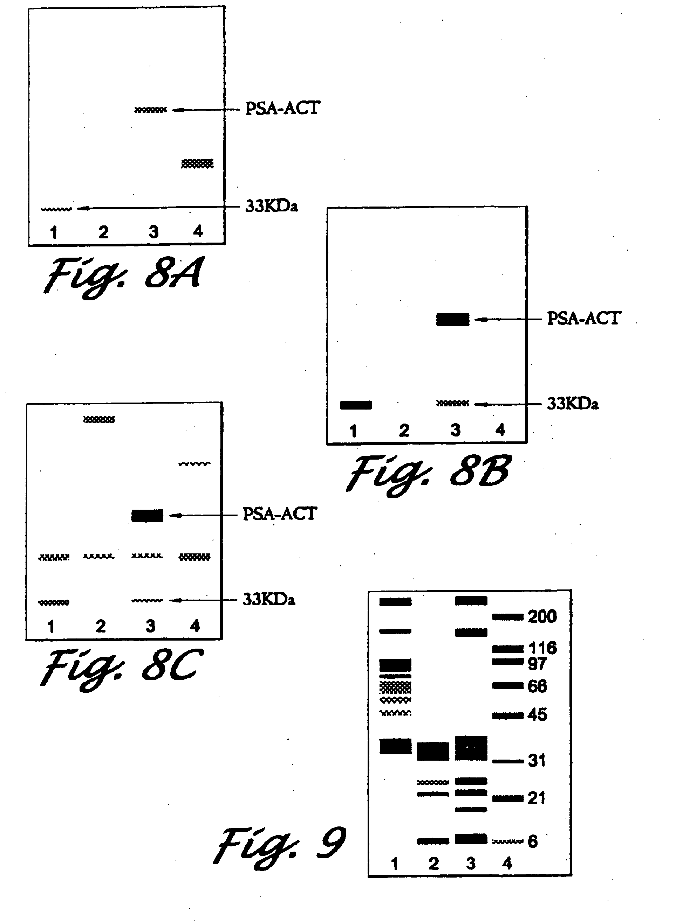

The anti-PSA monoclonal antibody detects a major band at approximately 90 kDa which represents PSA-ACT in all three serum specimens, as shown in Figure 8. A minor band at approximately 33 kDa is also detected in two of the three serum specimens. This lower molecular weight form of PSA represents an inactive uncomplexed form of PSA since active PSA would be readily complexed with ACT. The presence of the 33 kDa form of PSA in the serum samples with higher PSA levels probably is a reflection of the sensitivity of the method and antibody.

### EXAMPLE 5

### Clipped Forms of PSA are Not Detectable In Human (Prostate Cancer) Serum

### Methods:

### Western Blot of Serum

Reduced and denatured proteins were electrophoresed on 4-20% polyacrylamide gels and blotted onto nitrocellulose (Towbin, 1979). Successful transfer was checked by staining blots with Ponceau S protein stain. Blots were blocked in 5% nonfat dry milk in TRIS buffered saline (TBS) and then incubated with the anti-PSA monoclonal antibody PF1D215.1 at 0.1 µg/ml for 1 hour at 4°C with shaking. Following extensive washing with TBS, 0.05% tween, a 1:2,000 dilution of goat anti-mouse IgG-HRP in 5% milk (Jackson Laboratories, Westgrove, PA, USA) was incubated with the blots for 0.5 hour with shaking. Blots were washed five times with TBS and antibody reactivity was detected using the enhanced chemiluminescence (ECL) reagent (Amersham, Arlington Heights, IL, USA) according to manufacturer's instructions. The blot was scanned with the Chemilmager Low Light Imaging System (Alpha Innotech Corporation, San Leandro, CA, USA.).

Although the presence of clipped forms of PSA in seminal fluid is well-documented, the presence of these clipped forms has never been demonstrated in human serum, and has only been inferred as contributing to the proportion of PSA that exists in the uncomplexed, free form. Using an antibody that detects the clipped, breakdown products of PSA, PFI D 215, we examined prostate cancer serum for the presence of the breakdown products by Western blot analysis. In Figure 9, prostate cancer serum is compared to seminal fluid, each sample containing an equivalent amount of PSA. PSA purified from seminal fluid was examined as directly added to the gel and also as spiked into normal female serum. We demonstrate that, while the clipped/breakdown forms of PSA are evident in the seminal fluid samples (lane 3 - unspiked, lane 4 - spiked into female serum), there is no evidence of the clipped forms in human prostate cancer serum (lane 2). In addition, the predominant bands seen in the serum Western blot analysis are consistent with PSA-α2-macroglobulin complex, PSA-ACT complex, and an intact (unclipped) but inactive form of free PSA at 32 kd. This form is shown in Figure 6 to be, in part, pro-PSA and, in part, another (as yet uncharacterized) form of inactive PSA. These data demonstrate that free PSA in serum and seminal fluid are comprised of different molecular species.

## Claims

1. A method for detecting or determining pPSA in a sample of human physiological fluid containing pPSA comprising:
(a) providing an amount of purified antibodies which specifically bind a pPSA selected from [-1]pPSA, [-2]pPSA, [-3]pPSA, [-4]pPSA, [-5]pPSA, [-6]pPSA and [-7]pPSA and which do not display significant binding to the mature form of PSA, wherein the antibodies are raised against a recombinant pPSA expressed by a mammalian cell;
(b) contacting said antibodies with the sample to be tested for a sufficient time to allow the formation of binary complexes between at least a portion of said antibodies and a portion of said pPSA; and
(c) detecting or determining the presence or amount of pPSA complexed with said antibodies.

2. The method of Claim 1 wherein the human physiological fluid is serum.

3. The method of Claim 1 wherein said antibodies are monoclonal antibodies.

4. The method of Claim 1 wherein, in step (c), the pPSA is reacted with an antibody which comprises a detectable label or binds to a detectable label, to form a detectable ternary complex.

5. The method of Claim 4 wherein said antibodies are monoclonal antibodies.

6. The method of Claim 1 wherein said antibodies are attached to a solid phase.

7. A competition method for detecting or determining pPSA in a sample of human physiological fluid containing pPSA, comprising:
(a) providing an amount of purified antibodies which react with a pPSA selected from [-1]pPSA, [-2]pPSA, [-3]pPSA, [-4]pPSA, [-5]pPSA, [-6]pPSA and [-7]pPSA and which do not display significant reactivity to the mature form of PSA, wherein the antibodies are raised against a recombinant pPSA expressed by a mammalian cell;
(b) mixing the sample to be tested with a known amount of a pPSA or an immunoreactive subunit thereof that binds to an antibody which binds to said pPSA, which comprises a detectable label, to produce a mixed sample;
(c) contacting said antibodies with said mixed sample for a sufficient time to allow immunologic reactions to occur between said antibody and said pPSA in said sample and between said antibody and said labelled pPSA;
(d) separating the antibody from the mixed sample;
(e) detecting or determining the presence or amount of labelled pPSA either bound to the antibody or remaining in the mixed sample; and
(f) determining from the result in step (e) the presence or amount of said pPSA in said sample.

8. A method for detecting or determining pPSA in a mammalian tissue sample, comprising:
(a) mixing an amount of an agent which binds to a pPSA polypeptide selected from [-1]pPSA, [-2]pPSA, [-3]pPSA, [-4]pPSA, [-5]pPSA, [-6]pPSA and [-7]pPSA and which does not display significant binding to the mature form of PSA, with the cells of a mammalian tissue sample so as to form a binary complex comprising the agent and the cells wherein the agent comprises an antibody raised against a recombinant pPSA expressed by a mammalian cell; and
(b) determining or detecting the presence or amount of complex formation in the sample.

9. The method of Claim 8 wherein the antibody is a monoclonal antibody.

10. The method of Claim 8 wherein the cells are prostate cells.

11. The method of Claim 8 wherein complex formation is detected by a second agent comprising a detectable label or which binds to a detectable label, to form a detectable ternary complex.

12. The method of Claim 11 wherein the second agent is an antibody.

13. An antibody that binds a pro form of PSA selected from [-1]pPSA, [-2]pPSA, [-3]pPSA, [-4]pPSA, [-5]pPSA, [-6]pPSA and [-7]pPSA, and which does not display significant binding to the mature form of PSA, wherein the antibodies are raised against a recombinant pPSA, expressed by a mammalian cell.

14. The antibody of Claim 13 which does not display significant binding of the pro form of hK2.

15. An antibody that specifically binds [-4]pPSA and which does not display significant binding to the mature form of PSA.

16. The antibody of Claim 15 which does not display significant binding of [-5]pPSA or [-7]pPSA.

17. The antibody of Claim 15 which is a monoclonal antibody.

18. A hybridoma cell line producing an antibody which specifically binds pPSA selected from [-1]pPSA, [-2]pPSA, [-3]pPSA, [-4]pPSA, [-5]pPSA, [-6]pPSA and [-7]pPSA and which does not display significant binding to the mature form of PSA, wherein the antibody was raised against a recombinant pPSA expressed by a mammalian cell.

19. A hybridoma cell line producing an antibody which specifically binds [-4]pPSA and which does not display significant binding to the mature form of PSA.

20. A diagnostic kit for detecting or determining pPSA in human physiological fluid comprising a known amount of at least one antibody which specifically binds a pPSA selected from [-1]pPSA, [-2]pPSA, [-3]pPSA, [-4]pPSA, [-5]pPSA, [-6]pPSA and [-7]pPSA and which does not display significant binding to the mature form of PSA, wherein the antibody is detectably labelled or binds to a detectable label and wherein the antibodies are raised against a recombinant pPSA expressed by a mammalian cell.

21. The diagnostic kit of Claim 20 further comprising a solid phase capable of having said antibody attached thereto.

22. A diagnostic method comprising:
(a) contacting an amount of purified antibodies which specifically bind pPSA selected from [-1]pPSA, [-2]pPSA, [-3]pPSA, [-4]pPSA, [-5]pPSA, [-6]pPSA and
[-7]pPSA and which do not display significant binding to the mature form of PSA, with a sample of physiological fluid obtained from a human containing pPSA, for a sufficient time to allow the formation of binary complexes between at least a portion of said antibodies and a portion of said pPSA, wherein the antibodies are raised against a recombinant pPSA expressed by a mammalian cell; and
(b) determining the amount of said complexes in said sample and correlating the amount of said complexes to the presence or absence of prostate cancer in said human.

23. A method for distinguishing benign prostatic hyperplasia from prostate cancer comprising determining the presence or amount of pPSA selected from [-1]pPSA, [-2]pPSA, [-3]pPSA, [-4]pPSA, [-5]pPSA, [-6]pPSA, and [-7]pPSA in a sample of human physiological fluid wherein said determination does not use antibodies that display significant binding to the mature form of PSA.

## Patentansprüche

1. Verfahren zum Nachweis oder zum Bestimmen von pPSA in einer Probe menschlicher physiologischer Flüssigkeit, die pPSA enthält, umfassend
(a) das Bereitstellen einer Menge gereinigter Antikörper, die spezifisch ein pPSA, ausgewählt aus [-1]pPSA, [-2]pPSA, [-3]pPSA, [-4]pPSA, [-5]pPSA, [-6]pPSA und [-7]pPSA, binden, und die keine signifikante Bindung an die reife Form von PSA zeigen, wobei die Antikörper gegen ein rekombinantes pPSA, das von einer Säugerzelle exprimiert wurde, gerichtet sind;
(b) das Inkontaktbringen der Antikörper mit der zu testenden Probe für ausreichende Zeit, um die Bildung eines binären Komplexes zwischen zumindest einem Teil der Antikörper und einem Teil des pPSA ermöglicht; und
(c) das Nachweisen oder Bestimmen der Anwesenheit oder der Menge von pPSA, das mit den Antikörpern komplexiert ist.

2. Verfahren nach Anspruch 1, wobei die menschliche physiologische Flüssigkeit Serum ist.

3. Verfahren nach Anspruch 1, wobei die Antikörper monoklonale Antikörper sind.

4. Verfahren nach Anspruch 1, wobei in Schritt (c) das pPSA mit einem Antikörper reagieren gelassen wird, der eine nachweisbare Markierung umfaßt, oder an eine nachweisbare Markierung bindet, um einen nachweisbaren ternären Komplex zu bilden.

5. Verfahren nach Anspruch 4, wobei die Antikörper monoklonale Antikörper sind.

6. Verfahren nach Anspruch 1, wobei die Antikörper an eine feste Phase gebunden sind.

7. Kompetitionsverfahren zum Nachweis oder zum Bestimmen von pPSA in einer Probe menschlicher physiologischer Flüssigkeit, die pPSA enthält, umfassend
(a) das Bereitstellen einer Menge gereinigter Antikörper, die mit einem pPSA, ausgewählt aus [-1]pPSA, [-2]pPSA, [-3]pPSA, [-4]pPSA, [-5]pPSA, [-6]pPSA und [-7]pPSA, reagieren, und die keine signifikante Reaktivität mit der reifen Form von pPSA zeigen, wobei die Antikörper gegen ein rekombinantes pPSA, das von einer Säugerzelle exprimiert wurde, gerichtet sind;
(b) das Mischen der zu testenden Probe mit einer bekannten Menge eines pPSA oder einer immunreaktiven Untereinheit davon, die an einen Antikörper bindet, der das pPSA bindet, und der eine nachweisbare Markierung umfaßt, um eine gemischte Probe zu erzeugen;
(c) das Inkontaktbringen der Antikörper mit der gemischten Probe für eine Zeit, die ausreicht, um immunologische Reaktionen zwischen dem Antikörper und dem pPSA in der Probe und zwischen dem Antikörper und dem markierten pPSA geschehen zu lassen;
(d) das Abtrennen des Antikörpers von der gemischten Probe;
(e) das Nachweisen oder Bestimmen der Anwesenheit oder der Menge von markiertem pPSA, das entweder an den Antikörper gebunden ist oder in der gemischten Probe verbleibt; und
(f) das Bestimmen der Anwesenheit oder der Menge von pPSA in der Probe aus dem Ergebnis aus Schritt (e).

8. Verfahren zum Nachweis oder zum Bestimmen von pPSA in einer Säugergewebeprobe, umfassend
a) das Mischen einer Menge eines Mittels, das ein pPSA Polypeptid, ausgewählt aus [-1]pPSA, [-2]pPSA, [-3]pPSA, [-4]pPSA, [-5]pPSA, [-6]pPSA und [-7]pPSA, bindet, und das keine signifikante Bindung an die reife Form von PSA zeigt, mit den Zellen einer Säugergewebeprobe, um einen binären Komplex, umfassend das Mittel und die Zellen, zu bilden, wobei das Mittel einen Antikörper umfasst, der gegen ein rekombinantes pPSA, das von einer Säugerzelle exprimiert wurde, gerichtet ist; und
b) das Bestimmen oder den Nachweis der Anwesenheit oder der Menge an Komplexbildung in der Probe.

9. Verfahren nach Anspruch 8, wobei der Antikörper ein monoklonaler Antikörper ist.

10. Verfahren nach Anspruch 8, wobei die Zellen Prostatazellen sind.

11. Verfahren nach Anspruch 8, wobei die Komplexbildung durch ein zweites Mittel nachgewiesen wird, das eine nachweisbare Markierung umfaßt oder an eine nachweisbare Markierung bindet, um einen nachweisbaren ternären Komplex zu bilden.

12. Verfahren nach Anspruch 11, wobei das zweite Mittel ein Antikörper ist.

13. Antikörper der eine Vorform von PSA, ausgewählt aus [-1]pPSA, [-2]pPSA, [-3]pPSA, [-4]pPSA, [-5]pPSA, [-6]pPSA und [-7]pPSA, bindet, und der keine signifikante Bindung an die reife Form von pPSA zeigt, wobei der Antikörper gegen ein rekombinantes pPSA, das von einer Säugerzellen exprimiert wurde, gerichtet ist.

14. Antikörper nach Anspruch 13, der keine signifikante Bindung an die Vorform von hK2 zeigt.

15. Antikörper, der spezifisch [-4]pPSA bindet und der keine signifikante Bindung an die reife Form von PSA zeigt.

16. Antikörper nach Anspruch 15, der keine signifikante Bindung an [-5]pPSA oder [-7]pPSA zeigt.

17. Antikörper nach Anspruch 15, der ein monoklonaler Antikörper ist.

18. Hybridomzelllinie, die einen Antikörper erzeugt, der spezifisch pPSA, ausgewählt aus [-1]pPSA, [-2]pPSA, [-3]pPSA, [-4]pPSA, [-5]pPSA, [-6]pPSA und [-7]pPSA, bindet, und der keine signifikante Bindung der reifen Form von PSA zeigt, wobei die Antikörper gegen rekombinantes pPSA, das von einer Säugerzellen exprimiert wurde, gerichtet ist.

19. Hybridomzelllinie, die eine Antikörper erzeugt, der spezifisch [-4]pPSA bindet und der keine signifikante Bindung an die reife Form von PSA zeigt.

20. Diagnostisches Kit zum Nachweis oder zum Bestimmen von pPSA in menschlicher physiologischer Flüssigkeit, umfassend eine bekannte Menge von mindestens einem Antikörper, der spezifisch ein pPSA, ausgewählt aus [-1]pPSA, [-2]pPSA, [-3]pPSA, [-4]pPSA, [-5]pPSA, [-6]pPSA und [-7]pPSA, bindet, und der keine signifikante Bindung an die reife Form von PSA zeigt, wobei der Antikörper nachweisbar markiert ist oder an eine nachweisbare Markierung bindet, und wobei der Antikörper gegen ein rekombinantes pPSA, das von einer Säugerzelle exprimiert wurde, gerichtet ist.

21. Diagnostisches Kit nach Anspruch 20, weiterhin eine feste Phase umfassend, welche die Fähigkeit aufweist, den Antikörper an sich zu binden.

22. Diagnostisches Verfahren, umfassend
a) das Inkontaktbringen einer Menge gereinigter Antikörper, die spezifisch pPSA, ausgewählt aus [-1]pPSA, [-2]pPSA, [-3]pPSA, [-4]pPSA, [-5]pPSA, [-6]pPSA und [-7]pPSA, binden, und die keine signifikante Bindung an die reife Form von PSA zeigen, mit einer von einem Menschen erhaltenen Probe physiologischer Flüssigkeit, die pPSA enthält, für eine Zeit, die ausreicht, um die Bildung eines binären Komplexes zwischen mindestens einem Teil des Antikörpers und einem Teil des pPSA zu ermöglichen, wobei der Antikörper gegen rekombinante pPSA, das von einer Säugerzelle exprimiert wurde, gerichtet ist; und
b) das Bestimmen der Menge des Komplexes in der Probe und das Korrelieren der Menge des Komplexes mit der An- oder Abwesenheit von Prostatakrebs in dem Menschen.

23. Verfahren zum Unterscheiden von gutartiger Prostatahyperplasie von Prostatakrebs, umfassend das Bestimmen der Anwesenheit oder der Menge von pPSA, ausgewählt aus [-1]pPSA, [-2]pPSA, [-3]pPSA, [-4]pPSA, [-5]pPSA, [-6]pPSA und [-7]pPSA, in einer Probe menschlicher physiologischer Füssigkeit, wobei die Bestimmung keine Antikörper verwendet, die signifikante Bindung an die reife Form von PSA zeigen.

## Revendications

1. Méthode de détection ou de détermination du pPSA dans un échantillon d'un fluide physiologique humain contenant du pPSA comprenant :
(a) la fourniture d'une quantité d'anticorps purifiés qui se lient spécifiquement à un pPSA choisi parmi le [-1]pPSA, le [-2]pPSA, le [-3]pPSA, le [-4]pPSA, le [-5]pPSA, le [-6]pPSA et le [-7]pPSA et qui n'affichent pas de liaison significative avec la forme mature du PSA, dans laquelle les anticorps sont mobilisés contre un pPSA recombinant exprimé par une cellule de mammifère ;
(b) la mise en contact desdits anticorps avec l'échantillon qui doit être testé pendant un laps de temps suffisant pour permettre la formation de complexes binaires entre au moins une portion desdits anticorps et une portion dudit pPSA ; et
(c) la détection ou la détermination de la présence ou de la quantité de pPSA complexé avec lesdits anticorps.

2. Méthode selon la revendication 1, dans laquelle le fluide physiologique humain est le sérum.

3. Méthode selon la revendication 1, dans laquelle lesdits anticorps sont des anticorps monoclonaux.

4. Méthode selon la revendication 1, dans laquelle, dans l'étape (c), le pPSA est mis à réagir avec un anticorps qui comprend une marque détectable ou qui se lie à une marque détectable, afin de former un complexe ternaire détectable.

5. Méthode selon la revendication 4, dans laquelle lesdits anticorps sont des anticorps monoclonaux.

6. Méthode selon la revendication 1, dans laquelle lesdits anticorps sont attachés à une phase solide.

7. Méthode de compétition destinée à détecter ou à déterminer le pPSA dans un échantillon de fluide physiologique humain contenant le pPSA, comprenant :
(a) la fourniture d'une quantité d'anticorps purifiés qui réagissent avec un pPSA sélectionné parmi le [-1]pPSA, le [-2]pPSA, le [-3]pPSA, le [-4]pPSA, le [-5]pPSA, le [-6]pPSA et le [-7]pPSA et qui n'affichent pas de réactivité significative avec la forme mature de pPSA, dans laquelle les anticorps sont mobilisés contre un pPSA recombinant exprimé par une cellule de mammifère ;
(b) le mélange de l'échantillon qui doit être testé avec une quantité connue d'un pPSA ou d'une sous-unité immunoréactive de celui-ci qui se lie à un anticorps qui se lie au dit pPSA, qui comprend une marque détectable afin de produire un échantillon mixte ;
(c) la mise en contact desdits anticorps avec ledit échantillon mixte pendant un laps de temps suffisant pour permettre que des réactions immunologiques se produisent entre ledit anticorps et ledit pPSA dans ledit échantillon et entre ledit anticorps et ledit pPSA marqué ;
(d) la séparation de l'anticorps de l'échantillon mixte ;
(e) la détection ou la détermination de la présence ou de la quantité de pPSA marqué, soit liée à l'anticorps, soit restant dans l'échantillon mixte ; et
(f) la détermination, à partir du résultat de l'étape (e), de la présence ou de la quantité dudit pPSA dans ledit échantillon.

8. Méthode de détection ou de détermination de pPSA dans un échantillon de tissu de mammifère, comprenant :
(a) le mélange d'une quantité d'un agent qui se lie à un polypeptide de pPSA sélectionné parmi le [-1]pPSA, le [-2]pPSA, le [-3]pPSA, le [-4]pPSA, le [-5]pPSA, le [-6]pPSA et le [-7]pPSA et qui n'affiche pas de liaison significative avec la forme mature du PSA, avec les cellules d'un échantillon de tissu de mammifère de façon à former un complexe binaire comprenant l'agent et les cellules, dans laquelle l'agent comprend un anticorps mobilisé contre un pPSA recombinant exprimé par une cellule de mammifère ; et
(b) la détermination ou la détection de la présence ou de la quantité de formation complexe dans l'échantillon.

9. Méthode selon la revendication 8, dans laquelle l'anticorps est un anticorps monoclonal.

10. Méthode selon la revendication 8, dans laquelle les cellules sont des cellules de prostate.

11. Méthode selon la revendication 8, dans laquelle la formation complexe est détectée par un second agent comprenant une marque détectable ou qui se lie à une marque détectable pour former un complexe ternaire détectable.

12. Méthode selon la revendication 11, dans laquelle le second agent est un anticorps.

13. Anticorps qui se lie à une forme pro de PSA sélectionné parmi le [-1]pPSA, le [-2]pPSA, le [-3]pPSA, le [-4]pPSA, le [-5]pPSA, le [-6]pPSA et le [-7]pPSA et qui n'affiche pas de liaison significative avec la forme mature du PSA, dans laquelle les anticorps sont mobilisés contre un pPSA recombinant exprimé par une cellule de mammifère.

14. Anticorps selon la revendication 13, qui n'affiche pas de liaison significative à la forme pro de hK2.

15. Anticorps qui se lie spécifiquement au [-4]pPSA et qui n'affiche pas de liaison significative avec la forme mature du PSA.

16. Anticorps selon la revendication 15, qui n'affiche pas de liaison significative au [-5]pPSA ou au [-7]pPSA.

17. Anticorps selon la revendication 15, qui est un anticorps monoclonal.

18. Lignée cellulaire d'hybridome produisant un anticorps qui se lie spécifiquement au pPSA sélectionné parmi le [-1]pPSA, le [-2]pPSA, le [-3]pPSA, le [-4]pPSA, le [-5]pPSA, le [-6]pPSA et le [-7]pPSA et qui n'affiche pas de liaison significative avec la forme mature du PSA, dans laquelle l'anticorps a été mobilisé contre un pPSA recombinant exprimé par une cellule de mammifère.

19. Lignée cellulaire d'hybridome produisant un anticorps qui se lie spécifiquement au [-4]pPSA et qui n'affiche pas de liaison significative avec la forme mature du PSA.

20. Kit de diagnostic destiné à détecter ou à déterminer le pPSA dans un fluide physiologique humain comprenant une quantité connue d'au moins un anticorps qui se lie spécifiquement à un pPSA sélectionné parmi le [-1]pPSA, le [-2]pPSA, le [-3]pPSA, le [-4]pPSA, le [-5]pPSA, le [-6]pPSA et le [-7]pPSA et qui n'affiche pas de liaison significative avec la forme mature du PSA, dans laquelle l'anticorps est marqué de manière détectable ou se lie à une marque détectable, et dans laquelle les anticorps sont mobilisés contre un pPSA recombinant exprimé par une cellule de mammifère.

21. Kit de diagnostic selon la revendication 20 comprenant en outre une phase solide pouvant avoir ledit anticorps fixé sur celle-ci.

22. Méthode de diagnostic comprenant :
(a) la mise en contact d'une quantité d'anticorps purifiés qui se lient spécifiquement au pPSA sélectionné parmi le [-1]pPSA, le [-2]pPSA, le [-3]pPSA, le [-4]pPSA, le [-5]pPSA, le [-6]pPSA et le [-7]pPSA et qui n'affichent pas de liaison significative avec la forme mature du PSA, avec un échantillon de fluide physiologique obtenu chez un être humain contenant du pPSA, pendant un laps de temps suffisant pour permettre la formation de complexes binaires entre au moins une portion desdits anticorps et une portion dudit pPSA, dans laquelle les anticorps sont mobilisés contre un pPSA recombinant exprimé par une cellule de mammifère ; et
(b) la détermination de la quantité desdits complexes dans ledit échantillon et la mise en corrélation de la quantité desdits complexes avec la présence ou l'absence d'un cancer de la prostate chez ledit être humain.

23. Méthode permettant de distinguer l'hyperplasie prostatique bénigne du cancer de la prostate comprenant la détermination de la présence ou d'une quantité de pPSA sélectionné parmi le [-1]pPSA, le [-2]pPSA, le [-3]pPSA, le [-4]pPSA, le [-5]pPSA, le [-6]pPSA et le [-7]pPSA dans un échantillon d'un fluide physiologique humain, dans laquelle ladite détermination n'utilise pas des anticorps qui affichent une liaison significative avec la forme mature du PSA.
